# DEMANDE DE BREVET EUROPEEN

(11) **EP 0 645 126 A2**
(43) Date de publication de la demande: **29.03.1995**
(21) Numéro de dépôt: 94440047.2
(22) Date de dépôt: 21.07.1994
(51) Int. Cl.: A61F 2/38

(54) **Prothèse bicondylienne de l'articulation du genou postéro-stabilisée**

(30) Priorité: 21.07.1993 FR 9309149
(71) Demandeur: SOCIETE CIVILE ESSOR, F-74550 Perrignier (FR)
(72) Inventeur: Girou, Yves, F-85000 La Roche sur Yon (FR); Ducasse, Philippe, F-64000 Bayonne (FR)
(74) Mandataire: Polus, Camille

(57) **Abrégé**

Prothèse bicondylienne de l'articulation du genou de type dit à glissement, destinée à être mise en place en l'absence totale du ligament croisé postérieur, comprenant un implant fémoral (1) et un implant tibial (2), l'implant fémoral (1) comportant une barrette transversale intercondylienne (17) et l'implant tibial (2) comportant un ergot (43) saillant centralement du plateau tibial (4).

Ladite barrette transversale (17) de l'implant fémoral (1) est de section sensiblement circulaire ; la paroi verticale postérieure (44) de l'ergot (43) saillant du plateau tibial (4) est sensiblement perpendiculaire au plateau tibial (4), et le plateau tibial (4) est solidarisé à une embase (3) à l'aide de moyens d'ancrage (31) et de blocage latéral (32) et d'une vis (47) dont la partie filetée le traverse perpendiculairement par un orifice épaulé (49) pratiqué dans l'ergot (43) et se visse dans un orifice taraudé (34) pratiqué dans l'embase (3) de l'implant tibial (2)

## Description

La présente invention a pour objet une prothèse bicondylienne de l'articulation du genou du type dit à glissement, et plus particulièrement une prothèse dite postéro-stabilisée, destinée à être mise en place en l'absence totale du ligament croisé postérieur du genou.

Parmi les nombreux types de prothèses de l'articulation du genou connues à ce jour, celles dites à glissement sont celles qui reproduisent le mieux l'articulation naturelle, le fémur se terminant par deux condyles qui glissent sur le plateau tibial. Ce type de prothèse autorise notamment une rotation tibiale, comme celle décrite dans le document FR -A- 88 11666.

Toutefois, en l'absence du ligament croisé postérieur sectionné par accident, ce type de prothèse ne peut pas être implanté, car, tout comme dans l' articulation naturelle privée de ce ligament, il apparaîtrait un phénomène de tiroir, à savoir des mouvements anormaux d'avant en arrière dans le genou.

On implante alors généralement une prothèse monobloc à axe d'articulation, qui est toutefois moins fiable à l'usage que celles du type à glissement.

On connaît également des prothèses de l'articulation de genou dites postéro-stabilisées, par exemple celles décrites dans les documents FR-A-2.417.971, GB-A-2.067.412 et US-A-4.950.298.

Dans la prothèse objet du document FR-A-2.417.971, la postéro-stabilité est assurée par un plot saillant sensiblement centralement du plateau tibial et sur la face postérieure inclinée duquel glisse, lors de la flexion, une paroi transversale arrondie intercondylienne. L'inconvénient de cette prothèse réside dans la difficulté d'harmoniser ledit glissement avec le glissement des condyles sur le plateau tibial.

Dans le document GB-A-2.067.412 il est également proposé un plateau tibial comportant un plot central associé à une barrette transversale intercondylienne, qui n'entre toutefois en butée avec la paroi postérieure du plot central du plateau tibial qu'à partir d'un certain angle de flexion, laissant ainsi la prothèse fonctionner comme une prothèse non postéro-stabilisée pour des flexions d'angles moins importants.

Or, il arrive que la rupture ou le sectionnement des ligaments postérieurs croisés se produise au cours de l'opération de pose d'une prothèse non postéro-stabilisée, obligeant à remplacer cette dernière par une prothèse postéro-stabilisée alors que les os ont été sectionnés, ce qui peut être cause de difficultés importantes.

Le document US-A-4.950.298, en vue de remédier à ces inconvénients, propose une prothèse modulaire pouvant ou non être postéro-stabilisée selon les modules utilisés.

Toutefois cette prothèse présente comme inconvénient, d'une part qu'en configuration de prothèse postéro-stabilisée elle comporte beaucoup de pièces rapportées, et d'autre part que le bord de contact de la barrette transversale intercondylienne est plate et que la paroi postérieure du plot central est inclinée, en sorte que la flexion est limitée à une certaine valeur, la barrette étant plus une butée qu'un axe de rotation.

La présente invention vise à remédier aux inconvénients des prothèses connues en proposant une prothèse de l'articulation du genou du type à glissement, implantable en l'absence de ligament croisé postérieur.

La prothèse du genou selon la présente invention comprend, de manière connue en soi :
- un implant fémoral pourvu de deux condyles
- un implant tibial comportant une embase à quille, sur lequel est fixé de façon amovible un plateau en matière plastique du type polyéthylène ;
- un implant rotulien.

Pour pallier l'absence de ligament croisé postérieur, et éviter le phénomène de tiroir tout en permettant une flexion normale, la prothèse de l'articulation du genou selon l'invention présente en outre les caractéristiques suivantes :
- d'une part les condyles de l'implant fémoral sont prolongés intérieurement, de part et d'autre de l'échancrure intercondylienne, par deux flasques parallèles dont les bords supérieurs sont reliés, dans la région postérieure, par une barrette transversale de section sensiblement ronde ;
- d'autre part le plateau de l'implant tibial comporte en son milieu un ergot présentant en région postérieure une paroi transversale qui lui est sensiblement perpendiculaire, et comportant deux parois latérales parallèles, qui lui sont également sensiblement perpendiculaires.

Lorsque la prothèse selon l'invention est implantée, l'ergot du plateau tibial prend place dans l'échancrure intercondylienne de l'implant fémoral, entre les deux flasques et la barrette transversale.

Lors d'une flexion du genou, la barrette transversale entre en contact avec la paroi postérieure de l'ergot central du plateau tibial et devient l'axe de rotation de l'implant fémoral tout en maintenant le contact de glissement des condyles postérieurs avec le plateau tibial.

D'autre part les flasques latéraux de l'implant fémoral, en association avec les parois parallèles de l'ergot central du plateau tibial, limitent les déplacements transversaux de l'implant fémoral par rapport au plateau tibial, un certain jeu subsistant toutefois entre les flasques latéraux et l'ergot central de manière à autoriser une légère rotation tibiale.

La prothèse postéro-stabilisée selon la présente invention ne diffère de la prothèse bicondylienne à glissement décrite dans le document FR -A- 88 11666 que par la partie intercondylienne, ce qui autorise l'utilisation de la même instrumentation ancillaire et permet au chirurgien, lorsque les recoupes osseuses ont été réalisées, de choisir le type de prothèse, postéro-stabilisée ou non, qu'il va implanter sur son patient.

Selon un mode de réalisation avantageux de la prothèse selon l'invention, les surfaces d'appui des condyles sur le plateau tibial sont plates.

En effet, l'ergot central permet l'auto-centrage des condyles, ce qui permet la mise en oeuvre de surfaces d'appui planes qui assurent un meilleur glissement et donc un plus grand confort pour l'utilisateur par rapport aux prothèses existantes qui, pour permettre un auto-centrage des condyles sur le plateau tibial comportent des surfaces d'appui concaves.

Les avantages et les caractéristiques de la présente invention ressortiront plus clairement de la description qui suit et qui se rapporte au dessin annexé, lequel en représente un mode de réalisation non limitatif.

Dans le dessin annexé :
- la figure 1 représente une vue postérieure en perspective et en éclaté d'une prothèse de l'articulation du genou selon l'invention.
- les figures 2 représentent des vues de profil et en coupe selon un plan vertical médian de la même prothèse vue selon des angles de flexion différents, à savoir :
- la figure 2a en hyperextension à 14°
- la figure 2b en extension à 0°
- la figure 2c en flexion à 75°
- la figure 2d en flexion à 90°
- la figure 2e en flexion à 130°.

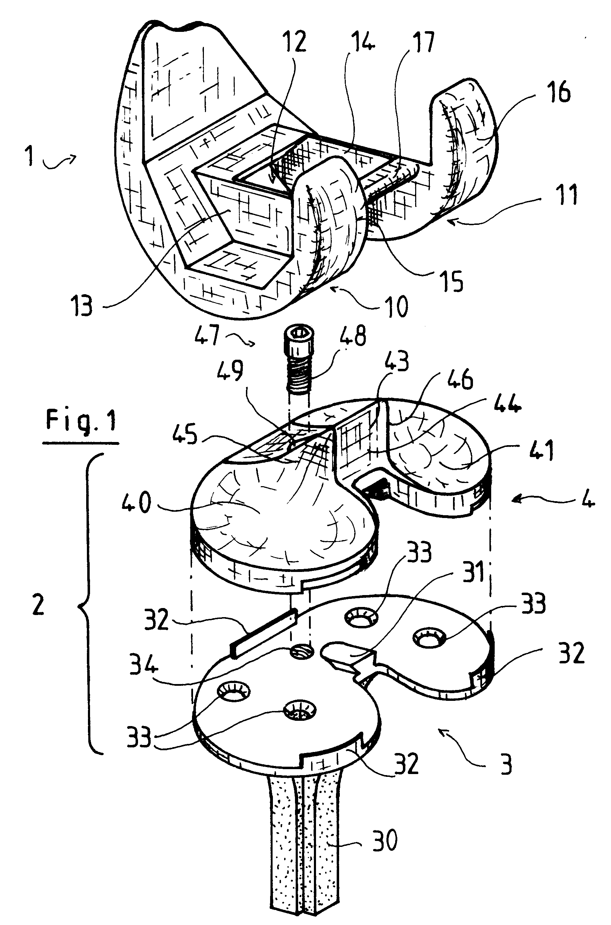

Si on se réfère à la figure 1 on peut voir qu'une prothèse de genou selon l'invention comporte d'une part un implant fémoral 1 et d'autre part un implant tibial 2 comprenant une embase tibiale 3 à quille 30 et un plateau tibial 4. L'implant rotulien de la prothèse n'est pas représenté car semblable à ceux existants .

L'implant fémoral 1 comporte deux condyles 10 et 11 prolongés chacun intérieurement, de part et d'autre de l'échancrure intercondylienne 12, par un flasque, respectivement 13 et 14, les flasques 13 et 14 étant parallèles et étant reliés, du côté des condyles postérieurs 15 et 16, par une barrette transversale 17, de section sensiblement ronde, solidarisée à leurs bords supérieurs.

Le plateau tibial 4 comporte en son milieu, entre les deux faces d'appui creuses 40 et 41, qui peuvent également être plates_et qui sont destinées à recevoir en appui les condyles 10 et 11, un ergot 43 en forme approximativement de coin, présentant postérieurement une paroi 44 et latéralement deux parois parallèles 45 et 46, toutes sensiblement perpendiculaires au plateau tibial 4. La largeur de l'ergot 43, entre les parois parallèles 45 et 46, est légèrement inférieure à la largeur de l'échancrure intercondylienne 12 de l'implant fémoral 1 bordée par les flasques latéraux 13 et 14, afin de permettre une insertion aisée de l'ergot 43 entre les flasques latéraux 13 et 14 lorsque l'implant fémoral 1 est mis en place sur le plateau tibial 4, et de permettre également une légère rotation tibiale.

L'embase 3 de l'implant tibial comporte des moyens, connus en soi, d'ancrage 31 et de calage latéral 32 du plateau tibial 4. Outre des perçages 33 permettant sa solidarisation au tibia au moyen de vis, non représentées, l'embase 3 comporte, dans l'axe de la quille 30, un orifice taraudé 34 permettant le vissage d'une vis 47 dont la partie filetée 48 traverse perpendiculairement le plateau tibial 4 par un orifice épaulé 49 pratiqué dans l'ergot central 43.

Il convient de remarquer que la fixation peut être biologique, la quille 30 et la face inférieure du plateau tibial 3 étant alors recouvertes d'hydroxyapatite et solidarisées au moyen d'un ciment acrylique, auquel cas le plateau tibial ne comporte pas de perçages 33.

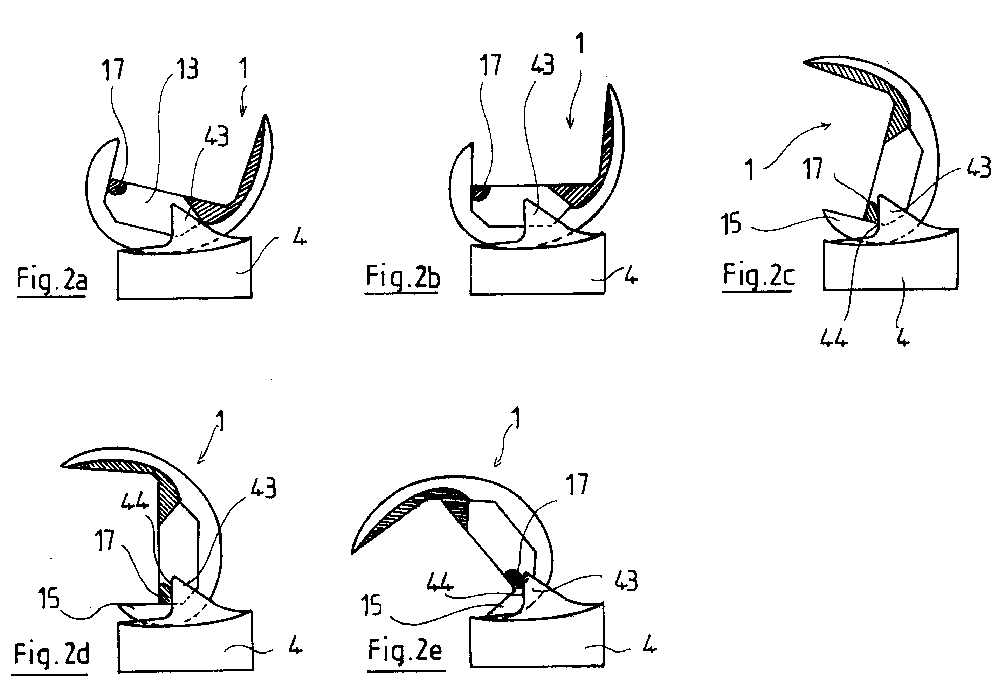

Si on se réfère maintenant aux figures 2 on peut voir la position de l'implant fémoral 1 par rapport au plateau tibial 4 selon l'angle de flexion de la jambe.

Sur les figures 2a et 2b on peut voir qu'en hyper extension et en extension l'implant fémoral 1 est autocentré par la courbure des faces d'appui du plateau tibial 4.

Sur les figures 2c, 2d et 2e on peut voir qu'une rotation associée à une légère translation antérieure de l'implant fémoral 1 sur le plateau tibial 4 s'opère jusqu'à ce que la barrette transversale 17 entre en contact avec la paroi transversale 44 de l'ergot central 43, cette limite étant atteinte pour une valeur approximative de 65° de flexion.

Entre cette valeur de 65° et la valeur maximum d'environ 130°, la barrette transversale 17 devient l'axe de rotation de l'implant fémoral 1 tout en maintenant le contact de glissement des condyles postérieurs 15 et 16 avec le plateau tibial 4.

Il va de soi que la présente invention ne saurait être limitée à la description qui précède d'un de ses modes de réalisation, susceptible de subir un certain nombre de modifications sans pour autant sortir du cadre de l'invention.

## Revendications

**1)** Prothèse bicondylienne de l'articulation du genou de type dit à glissement, destinée à être mise en place en l'absence totale du ligament croisé postérieur, comprenant un implant fémoral (1) et un implant tibial (2), l'implant fémoral (1) comportant une barrette transversale intercondylienne (17) et l'implant tibial (2) comportant un ergot (43) saillant centralement du plateau tibial (4), caractérisée d'une part en ce que ladite barrette transversale (17) de l'implant fémoral (1) est de section sensiblement circulaire ; d'autre part en ce que la paroi verticale postérieure (44) dudit ergot (43) saillant du plateau tibial (4) est sensiblement perpendiculaire audit plateau tibial (4), et d'autre part encore en ce que ledit plateau tibial (4) est solidarisé à une embase (3) à l'aide de moyens d'ancrage (31) et de blocage latéral (32) et d'une vis (47) dont la partie filetée traverse perpendiculairement ledit plateau tibial (4) par un orifice épaulé (49) pratiqué dans l'ergot (43), et se visse dans un orifice taraudé (34) pratiqué dans l'embase (3) dudit implant tibial (2).

**2)** Prothèse selon la revendication 1 caractérisé en ce que les surfaces d'appui (40, 41) des condyles (10, 11) sur le plateau tibial (4) sont plates.
